# EUROPÄISCHE PATENTSCHRIFT

(11) **EP 3 117 806 B1**
(45) Veröffentlichungstag und Bekanntmachung des Hinweises auf die Patenterteilung: **10.06.2020**
(21) Anmeldenummer: 15002120.2
(22) Anmeldetag: 16.07.2015
(51) Int. Cl.: A61F 13/00, A61M 1/00, A61F 13/06, A61F 13/08, A61F 13/10, A61F 13/02

(54) **WUNDVERSORGUNGSANORDNUNG**
WOUND TREATMENT ASSEMBLY
SYSTEME DE TRAITEMENT DES PLAIES

(43) Veröffentlichungstag der Anmeldung: 18.01.2017
(73) Patentinhaber: Lohmann & Rauscher GmbH, 2525 Schönau/Triesting (AT)
(72) Erfinder: Schneider, Johannes, 80801 München (DE)
(74) Vertreter: Seranski, Klaus

(56) Entgegenhaltungen:
- EP-A1- 2 636 417
- WO-A1-2010/011148
- WO-A1-2015/022334
- WO-A2-2007/002835
- DE-A1-102011 106 540
- DE-A1-102012 205 408
- GB-A- 2 329 127
- US-A1- 2006 149 171

## Beschreibung

Die Erfindung betrifft eine Wundversorgungsanordnung mit einer an der eine Wunde umgebenden Haut festlegbaren und zum Herstellen eines die Wunde enthaltenden abgeschlossenen Wundraums dienenden, zumindest abschnittweise wasserdampfdurchlässigen und einen Folienschlauch aufweisenden Abdeckeinrichtung.

Derartige Wundversorgungsanordnungen werden insbesondere im Rahmen der sogenannten Vakuumtherapie eingesetzt. Es hat sich gezeigt, dass im Besonderen die Heilung chronischer Wunden durch Anlegen von Unterdruck an diese Wunden gefördert werden kann. Dabei hat es sich weiter als vorteilhaft erwiesen, wenn die Wunde mit einem offenporigen Schaum oder Gaze als Füllmaterial abgedeckt bzw. gefüllt wird, die Wunde zur Erzeugung eines die Wunde und gegebenenfalls das Füllmaterial enthaltenden abgeschlossenen Wundraums abgedeckt und auf der der Wunde bzw. dem Füllmaterial abgewandten Seite der Abdeckeinrichtung ein Sauganschluss angebracht wird, über den der Wundraum mit einer zum Erzeugen von Unterdruck ausgelegten Saugeinrichtung verbunden werden kann. Bei anderen Anordnungen wird ein Flansch des Sauganschlusses von der Abdeckeinrichtung abgedeckt oder in einer eine Öffnung der Abdeckeinrichtung umlaufenden Tasche aufgenommen. Der Sauganschluss kann beispielsweise mit einem einerseits an einer beispielsweise in Form eines Rohrstutzens ausgeführten Verbindungseinrichtung des Sauganschlusses und andererseits mit einem an eine Saugeinrichtung anschließbaren Schlauch ausgestattet sein. Die Abdeckeinrichtung kann beispielsweise als folienartiges Material ausgeführt sein, welches an die der Wunde benachbarten Hautfläche luftdicht angelegt wird.

Wundversorgungsanordnungen, die im Rahmen der Vakuumtherapie einsetzbar sind, sind beispielsweise in der EP 0 620 720 B1 beschrieben. Der Offenbarungsgehalt dieser Schrift wird hinsichtlich der Einzelheiten der im Rahmen der Vakuumtherapie einsetzbaren schaumbildenden Mittel und der Saugeinrichtung hiermit durch ausdrückliche Inbezugnahme in diese Beschreibung aufgenommen. Diese Einzelheiten können in Kombination mit den Merkmalen des Hauptanspruchs zur Lösung der dieser Erfindung zugrunde liegenden Aufgabe beitragen. Auch für solche Kombinationen wird Schutz begehrt.

In der DE 10 2009 019 646 A1 ist ein zur Verbesserung des Exsudat-Management zwischen dem Füllmaterial und dem Wundgrund einzulegende Kontaktschicht beschrieben, die einen Drainageraum zwischen dem Füllmaterial und dem Wundgrund bildet. Der Offenbarungsgehalt dieser Schrift wird ausdrücklich in diese Beschreibung aufgenommen. Diese in dieser Schrift beschriebenen Einzelheiten hinsichtlich der den Drainageraum bildenden Kontaktschicht bzw. Wundabdeckung können in Kombination mit den Merkmalen des Hauptanspruchs zur Lösung der dieser Erfindung zugrunde liegenden Aufgabe beitragen. Auch für solche Kombinationen wird Schutz begehrt.

Im Rahmen der Vakuumtherapie einsetzbare Sauganschlüsse, welche über einen Schlauch mit einer Saugeinrichtung verbunden werden können, sind beispielsweise in der WO 03/073970 A1, der WO 2008/014358 A2 und der WO 2009/124548 A1 beschrieben. Ein als Saugkopf bezeichneter Sauganschluss mit zur Strömungsführung im Bereich der der Wunde zugewandten Begrenzungsfläche des Sauganschlusses dienenden Vorsprüngen ist in der EP 1 018 967 B1 beschrieben. Ferner ist ein Sauganschluss mit einer an das Füllmaterial anzulegenden Anlagefläche in Form einer scheibenartigen Schale in der EP 1 088 569 B1 angegeben. Bei einem in der WO 2010/008167 A2 beschriebenen Sauganschluss sind auf der dem Füllmaterial zugewandten Begrenzungsfläche durch Stege begrenzte Kanäle gebildet, durch die das Wundexsudat in Richtung auf eine Absaugöffnung geleitet werden soll.

In der WO 2010/011148 A1 ist eine im Rahmen der Vakuumtherapie einsetzbare Wundversorgungsanordnung beschrieben, die einen über eine Extremität des menschlichen Körpers ziehbaren undurchlässigen Schlauch sowie einen zwischen der Wunde und dem Schlauch anzuordnenden perforierten Körper aufweist. Mit dem perforierten Körper wird zwischen dem undurchlässigen Schlauch und dem Wundgrund ein Raum geschaffen, in dem über einen dichtend an den undurchlässigen Schlauch anlegbaren Schlauchanschluss ein Unterdruck erzeugt werden kann.

In der EP 1 162 932 B1 ist eine Wundversorgungsanordnung mit einer Umhüllung aus einem Kunststoffmaterial und einem in der Umhüllung enthaltenen fluidabsorbierenden Material beschrieben. Die in dieser Schrift beschriebene Wundversorgungsanordnung ist zum Schutz von Wunden gedacht. Sie ist mangels Schlauchanschluss für den Einsatz in der Vakuumtherapie nicht geeignet.

In der EP 2 636 417 A1 ist eine Wundversorgungsanordnung nach dem Oberbegriff des Patentanspruchs 1 angegeben. Die Wundversorgungsanordnung kann einen wasserdampfdurchlässigen Folienschlauch aufweisen, der zur Behandlung von Wunden an den Extremitäten, z.B. Fuß, Knöchel, Unterarm, Arm, Hand, über die Extremität gezogen und bezüglich der Wunde so positioniert wird, dass die Wunde mit der Abdeckeinrichtung dichtend abgedeckt wird. Im Anschluss daran kann die Abdeckeinrichtung mit der Haftfolie an der der Wunde benachbarten Haut befestigt werden. Dazu kann es vorgesehen sein, dass die Haftfolie von einem Wickel abgezogen und derart um ein Ende des Schlauchs gewickelt wird, dass sie einerseits an der Abdeckeinrichtung und andererseits an der Haut haftet. Der Offenbarungsgehalt der EP 2 636 417 A1 wird hinsichtlich der Eigenschaften von wasserdampfdurchlässigen Abdeckfolien, Haftfolien und der Verfahren zum Anlegen von Wundversorgungsanordnungen hiermit durch ausdrückliche Inbezugnahme in diese Beschreibung aufgenommen. Diese Einzelheiten können in Kombination mit den Merkmalen des Hauptanspruchs zur Lösung der dieser Erfindung zugrunde liegenden Aufgabe beitragen. Auch für solche Kombinationen wird Schutz begehrt.

Bei Einsatz der in der EP 2 636 417 A1 beschriebenen Wundversorgungsanordnung zur Wundbehandlung im Bereich der Extremitäten, im Besonderen im Bereich des Oberarms oder Oberschenkels hat es sich gezeigt, daß die Erzeugung eines Unterdrucks im Wundraum in vielen Fällen Schwierigkeiten bereitet.

Weitere Wundversorgungsanordnungen sind in der US 2006/149171 A1, der DE 10 2011 106 540 A1, der WO 2015/022334 A1, der GB 2 329 127 A, der DE 10 2012 205 408 A1 und der WO 2007/002835 A2 angegeben.

Angesichts dieser Probleme im Stand der Technik liegt der Erfindung die Aufgabe zugrunde, eine Wundversorgungsanordnung anzugeben, mit der die Vakuumtherapie auch im Bereich der Oberarme und/oder Oberschenkel erfolgreich eingesetzt werden kann.

Erfindungsgemäß wird diese Aufgabe durch die im kennzeichnenden Teil des Patentanspruchs 1 angegebene Wundversorgungsanordnung gelöst, die im Wesentlichen dadurch gekennzeichnet ist, daß der Folienschlauch über einen eine quer, insbesondere etwa senkrecht zu einer durch die Schlauchachse und eine die Schlauchachse durchsetzende und senkrecht dazu verlaufende Radialachse aufgespannte Frontalebene verlaufende Sagittalachse zumindest teilweise umlaufenden Übergangsbereich in einen Haltelappen übergeht.

Dabei geht die Erfindung auf die Erkenntnis zurück, daß die im Stand der Technik beobachteten Probleme maßgeblich auf die mangelhafte Abdichtung im Bereich des dem Rumpf zugewandten Endes des Oberarms oder Oberschenkels bei Bewegung der entsprechenden Extremität, aber auch bei der Applikation und dem dichtenden Anschluss, zurückzuführen sind. Der Übergang des Oberarms zum Rumpf wird durch die Achselhöhle gebildet, welche bei Bewegungen des Arms Falten bildet, durch die der dichtende Anschluss der Abdeckeinrichtung an der der Wunde benachbarten Haut beeinträchtigt wird. Ähnlich wird der dichtende Abschluss der Abdeckeinrichtung im Bereich des den Übergang vom Oberschenkel zum Rumpf bildenden Schritts durch Faltenbildung bei Bewegungen, aber auch bei der Applikation und dem dichtenden Anschluss,beeinträchtigt.

Mit erfindungsgemäßen Wundversorgungsanordnungen wird der Folienschlauch um einen Übergangsbereich und einen Haltelappen erweitert, mit denen Achselhöhle und/oder Schritt des Patienten flächig abgedeckt werden können, so daß ein dichtender Anschluss der Abdeckeinrichtung an der Haut des Patienten außerhalb dieser kritischen Bereiche vorgenommen werden kann. Überraschenderweise hat es sich gezeigt, daß die durch die Erweiterung der Abdeckeinrichtung und die damit einhergehenden Verlängerung der Ränder der Abdeckeinrichtung zu erwartende Beeinträchtigung des dichtenden Anschlusses an die Haut des Patienten so geringfügig ist, daß die mit der Erfindung erreichten Vorteile bei Weitem überwiegen und die erfolgreiche Anwendung der Vakuumtherapie auch im Bereich von Oberarm und Oberschenkel ermöglichen, wobei sogar im Bereich des Schritts und/oder der Achselhöhle vakuumtherapeutische Maßnahmen unter Einsatz erfindungsgemäßer Wundversorgungsanordnungen erfolgreich durchgeführt werden können.

Mit anderen Worten ausgedrückt, ermöglicht eine erfindungsgemäße Wundversorgungsanordnung also schwer abzudichtende Körperstellen, wie etwa zur Faltenbildung neigende Bereiche der Haut des Patienten, zu verschließen. Andererseits ist ein Verschluss von Wunden möglich, ohne die Haut mit klebenden Wundverbänden zu bekleben.

Im Rahmen der Erfindung wird eine Verbesserung des dichtenden Anschlusses der Abdeckeinrichtung an der Haut des Patienten erreicht, weil der Haltelappen einen sich ausgehend von den im kritischen Bereich der Achselhöhle und/oder des Schritts umlaufenden Übergangsbereich zumindest abschnittsweise in Richtung auf das vom Übergangsbereich abgewandte Ende des Folienschlauchs erstreckenden Halteabschnitt aufweist, der eine in der Frontalebene Longitudinalachse teilweise, insbesondere über einen Umfangswinkel von 180° oder weniger, und 45° oder mehr, umläuft, wobei die Longitudinalachse in einer die Schlauchachse enthaltenden und senkrecht zur Sagittalachse verlaufenden Ebene angeordnet ist.

Durch die Erweiterung der Abdeckeinrichtung um einen derartigen Halteabschnitt wird eine dichtende Befestigung des Halteabschnitts an solchen Körperbereichen des Patienten ermöglicht, die kaum zur Faltenbildung im Verlauf von Bewegungen neigen, wie etwa im Bereich des Oberschenkels und/oder des Brustbereichs des Patienten. Andererseits ermöglicht der nur teilweise Umlauf des Halteabschnitts um die Longitudinalachse eine erleichterte Applikation der Abdeckeinrichtung an den Patienten, weil der Folienschlauch nur über die betroffene Extremität gezogen werden muss und es nicht erforderlich ist, die andere Extremität ebenfalls vollständig durch die Abdeckeinrichtung zu führen, wie es bei einer hosen- oder jackenartigen Abdeckeinrichtung der Fall wäre.

Bei erfindungsgemäßen Wundversorgungsanordnungen kann die Schlauchachse (Achse der zu behandelnden Extremität) etwa parallel zur Longitudinalachse (Körperachse) verlaufen. Im Rahmen der Erfindung hat es sich jedoch mit Blick auf eine Vereinfachung der Applikation der Abdeckeinrichtung als vorteilhaft erwiesen, wenn die Schlauchachse und die Longitudinalachse einen spitzen Winkel von vorzugsweise etwa 2° bis 45°, insbesondere 5° bis 30°, besonders bevorzugt 5° bis 15° miteinander einschließen.

Im Sinne einer sicheren Abdeckung der kritischen Körperbereiche wie etwa der Achselhöhle oder des Schritts hat es sich als zweckmäßig erwiesen, wenn der dem Folienschlauch zugewandte Rand des Übergangsbereichs die Schlauchachse teilweise, vorzugsweise über einen Winkelbereich von 180° oder weniger und mehr als 20°, insbesondere mehr als 45°, vorzugsweise mehr als 60°, besonders bevorzugt mehr als 90° umläuft. Bei dieser Ausführungsform der Erfindung kann der Übergangsbereich zumindest teilweise als radial innenliegendes Segment eines Rotationstorus ausgeführt sein.

Ebenfalls im Sinne einer zuverlässigen Abdeckung kritischer Körperbereiche hat es sich als bevorzugt erwiesen, wenn der den Folienschlauch mit dem Haltelappen verbindende Übergangsbereich die Sagittalachse über einen Winkelbereich von 180° oder weniger und mehr als 45°, vorzugsweise mehr als 60°, insbesondere mehr als 75° umläuft.

Im Sinne eines dichten Anschlusses der Abdeckeinrichtung an die Haut des Patienten ohne Beeinträchtigung durch im Bereich der Achselhöhle und/oder des Schritts vermehrt auftretenden Hautfalten, hat es sich ferner als zweckmäßig erwiesen, wenn die Abdeckeinrichtung einer erfindungsgemäßen Wundversorgungsanordnung zwei von dem Übergangsbereich ausgehende und in sagittaler Richtung voneinander beabstandete Haltelappen vorgesehen sind. Dabei kann bei einer Versorgung des Arms einer der Haltelappen an der Brust des Patienten anliegen, während der andere am Rücken des Patienten anliegt. Bei Versorgung einer Wunde im Bereich des Oberschenkels kann einer der Haltelappen dorsal angeordnet sein, während der andere ventral angeordnet ist.

Vorzugsweise sind die Haltelappen an ihren dem Übergangsbereich abgewandten Rändern nicht miteinander verbunden. Vielmehr laufen sie an ihren den Übergangsbereichen abgewandten Enden in frei auslaufenden Rändern aus. Dadurch wird die Applikation der Abdeckeinrichtung erleichtert, weil die Haltelappen unabhängig voneinander dorsal und ventral an der Haut des Patienten dichtend angeschlossen werden können.

Im Rahmen der Erfindung ist allerdings auch an den Einsatz solcher Ausführungsformen gedacht, bei denen die dem Übergangsbereich abgewandten Ränder zumindest abschnittweise miteinander verbunden, insbesondere verklebt sind.

Eine weitere Verbesserung der dichtenden Befestigung der Abdeckeinrichtung an der Haut des Patienten kann erreicht werden, wenn der Folienschlauch auf seiner in Umfangsrichtung bezüglich der Schlauchachse dem Übergangsbereich abgewandten Seite in einen die Schlauchachse in Umfangsrichtung über einen Umfangswinkelbereich von 30° oder mehr, vorzugsweise 60° oder mehr, insbesondere 90° oder mehr, besonders bevorzugt etwa 180° - 220° umlaufenden Bundbereich übergeht. Bei Einsatz eines solchen Bundbereichs kann auch der Anschluss der Abdeckeinrichtung an die Haut des Patienten außerhalb des Haltelappens mit Abstand von den zur Faltenbildung neigenden Hautbereichen erfolgen.

Im Sinne einer Erleichterung der Applikation der Abdeckeinrichtung und zur Verbesserung der Dichteigenschaften der Abdeckeinrichtung erfindungsgemäßer Wundversorgungsanordnungen hat es sich als zweckmäßig erwiesen, wenn der Bundbereich in Umfangsrichtung in mindestens einen Haltelappen übergeht, wobei der dem Folienschlauch abgewandte Rand des Bundbereichs vorzugsweise mit einem die Longitudinalachse teilweise umlaufenden Rand des Haltelappens fluchtet.

Fertigungstechnisch und im Sinne einer einfachen Applikation hat es sich als vorteilhaft erwiesen, wenn der Folienschlauch, der Übergangsbereich, der Haltelappen und ggf. der Bundbereich ein zusammenhängendes, dreidimensionales Flächengebilde bilden.

Dieses dreidimensionale Flächengebilde kann im Sinne einer Vereinfachung des dichtenden Anschlusses der Abdeckeinrichtung an der Haut des Patienten so ausgeführt sein, daß von dem dem Übergangsbereich in Richtung der Schlauchachse abgewandten Rand des Folienschlauchs, dem dem Übergangsbereich in Richtung der Schlauchachse abgewandten Rands des Bundbereichs und Rändern des bzw. der Haltelappen vollständig berandet wird. Bei dieser Ausführung ist es nicht erforderlich, einen dichtenden Anschluss des Übergangsbereichs an die Haut des Patienten herzustellen. Der dichtende Anschluss kann dann insgesamt an Hautbereichen befestigt werden, die weniger zu Faltenbildung neigen.

Nachstehend wird die Erfindung unter Bezugnahme auf die Zeichnung, auf die hinsichtlich aller erfindungswesentlichen Merkmale, die in der Beschreibung nicht ausdrücklich erwähnt sind, Bezug genommen wird, erläutert. In der Zeichnung zeigt:
- Fig. 1: eine erste Ausführungsform einer erfindungsgemäßen Wundversorgungsanordnung und
- Fig. 2: eine zweite Ausführungsform einer erfindungsgemäßen Wundversorgungsanordnung.

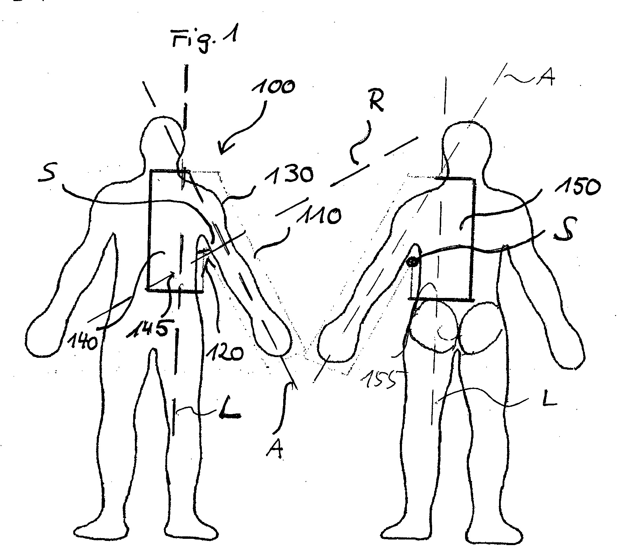

Die in Fig. 1 dargestellte Wundversorgungsanordnung 100 umfasst einen Folienschlauch 110, der über einen Übergangsbereich 120 in einen ventralen Haltelappen 140 und einen dorsalen Haltelappen 150 übergeht. Der dorsale Haltelappen 150 und der ventrale Haltelappen 140 sind nicht nur über den Übergangsbereich 120, sondern auch über den Bundbereich 130 mit dem Folienschlauch 110 verbunden. Der Folienschlauch 110 ist so dimensioniert, daß er über den Arm des Patienten gezogen werden kann. An seinem der Hand des Patienten abgewandten Ende geht der Folienschlauch 110 in den Bundbereich 130 über, der die Schlauchachse über eine Winkel von etwa 180° umläuft und dann in die Haltelappen 140 und 150 übergeht. Der ventrale Haltelappen 140 ist geradlinig berandet und kann mit Hilfe geeigneter Haftstreifen oder eines auf dem Lappen selbst aufgetragenen Haftmittels dichtend an die Haut des Patienten angeschlossen werden. Ebenso ist der dorsale Haltelappen 150 geradlinig berandet und kann über entsprechende Haftstreifen oder an dem Haltelappen selbst angebrachte Haftmittel dichtend an die Haut des Patienten angeschlossen werden.

Auf der dem Bundbereich 130 in Umfangsrichtung bezüglich der Schlauchachse abgewandten Seite geht der Folienschlauch 110 in den Übergangsbereich 120 über. Dieser umläuft eine senkrecht zu einer durch die Schlauchachse A und eine die Schlauchachse A durchsetzende und senkrecht dazu verlaufende Radialachse aufgespannte Frontalebene verlaufende Sagittalachse S über einen Umfangswinkel von etwa 320°. Im Anschluss geht der Übergangsbereich 120 in die Haltelappen 140 und 150 über, welche an ihrem dem Folienschlauch 110 zugewandten Rand im Bereich sich ausgehend vom Übergangsbereich120 in Richtung auf das dem Übergangsbereich 120 abgewandte Ende des Folienschlauchs angeordneten Halteabschnitts 145 bzw. 155 miteinander verbunden sind. An ihren dem Folienschlauch 110 abgewandten Rändern laufen der ventrale Haltelappen 140 und der dorsale Haltelappen 150 in freien Rändern aus. Insgesamt bilden der Folienschlauch 110, der Bundbereich 130, der Übergangsbereich 120 und die Haltelappen 140 und 150 ein durchgehendes Flächengebilde, das vollständig von den Rändem des Bundbereichs und der Haltelappen berandet wird, so daß nur in diesem Bereich ein dichtender Anschluss an die Haut des Patienten hergestellt werden muss. Im Besonderen im Bereich der Achselhöhle muss ein solcher dichtender Anschluss nicht hergestellt werden, da dieser Bereich vom Übergangsbereich und den Haltelappen vollständig und lückenlos abgedeckt wird.

Der Folienschlauch 120 umläuft eine Schlauchachse A in Umfangsrichtung vollständig. Ausgehend von einem der Hand abgewandten Rand des Folienschlauchs 110 umläuft der Übergangsbereich 120 eine Sagittalachse S über einen Winkelbereich von etwa 160°. Die Haltelappen 140 und 150 umlaufen eine in der Frontalebene verlaufende Longitudinalachse L, die in einer senkrecht zur Sagittalachse S und die Schlauchachse A enthaltenen Ebene angeordnet ist. Dabei umlaufen die Haltelappen 140 und 150 die Longitudinalachse insgesamt nur über einen Umfangswinkel von etwa 180°. Die Halteabschnitte 145 und 155 der Halte-lappen 140 und 150 sind an ihren dem Folienschlauch 110 zugewandten Rändern miteinander verbunden. Demnach ist der Übergangsbereich 120 einerseits über einen Umfangswinkelbereich von etwa 180° bzgl. der Schlauchachse A mit dem Folienschlauch 110 verbunden, während der andere über einen Umfangswinkelbereich von 180° bzgl. der Longitudinalachse L mit den Haltelappen 140 bzw. 145 verbunden ist.

Der Sauganschluss der Wundversorgungsanordnung ist in Fig. 1 nicht dargestellt. Er kann in der Nähe des Wundbereichs angeordnet sein. Regelmäßig muss er der jeweiligen Behandlung angepasst werden. Dabei wird er üblicherweise über der Wunde platziert, häufig zentral über der Wunde, aber je nach Lokalisation der Wunde auch an anderer Position über der Wunde. Dabei ist zwischen dem Wundgrund und der Abdeckeinrichtung zweckmäßigerweise auch noch ein Füllmaterial, wie etwa ein Füllschaum, Gaze, oder dergleichen vorgesehen. Erkennbar ist, daß bei der in Fig. 1 dargestellten Ausführungsform der Erfindung die Schlauchachse und die von den Haltelappen 140 und 150 umlaufende Longitudinalachse einen spitzen Winkel von etwa 20° miteinander einschließen.

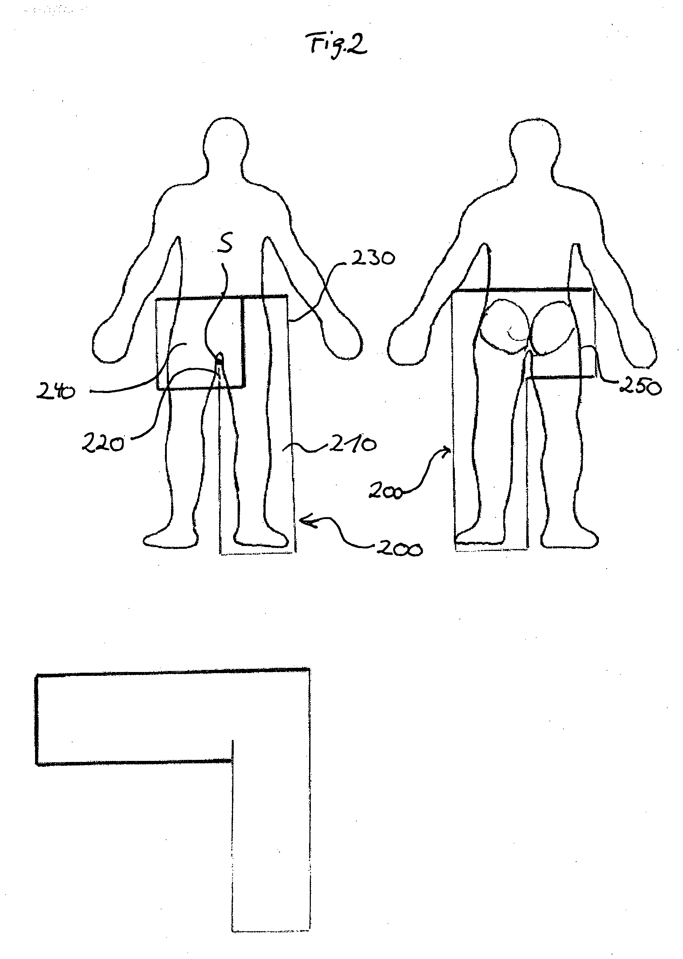

Die in Fig. 2 dargestellte Ausführungsform der Erfindung umfasst ebenso wie die anhand der Fig. 1 erläuterte Ausführungsform eine Abdeckeinrichtung 200 mit einem Folienschlauch 210, einem Übergangsbereich 220, einem Bundbereich 230, einem ventralen Haltelappen 240 und einem dorsalen Haltelappen 250. Der Folienschlauch 210 der in Fig. 2 dargestellten Ausführungsform ist so dimensioniert, daß er über ein Bein des Patienten gezogen werden kann. Der Übergangsbereich 220 umläuft eine den Schritt des Patienten durchsetzende Sagittalachse S um einen Umfangswinkel von etwa 180°. Die Haltelappen 240 und 250 umlaufen eine parallel zur Schlauchachse verlaufende Longitudinalachse. Sie sind dichtend mit dem den Folienschlauch auf seiner den Haltelappen 240 und 250 in Umfangsrichtung bezüglich der Schlauchachse abgewandten Seite verlängernden Bundbereich 230 verbunden. Die Haltelappen 240 und 250 sind ebenso wie in der anhand der in Fig. 1 dargestellten Ausführungsform der Erfindung geradlinig berandet und können längs ihrer Ränder dichtend an die Haut des Patienten angeschlossen werden. Die Applikation der anhand der Fig. 2 erläuterten Abdeckeinrichtung wird dadurch erleichtert, daß die Haltelappen an ihren dem Übergangsbereich 220 abgewandten Ende frei auslaufen, so daß die Haltelappen unabhängig voneinander an die Haut des Patienten angeschlossen werden können.

Die Erfindung ist nicht auf die anhand der Zeichnung erläuterten Ausführungsbeispiele eingeschränkt. Wesentlich zum Erhalt der mit der Erfindung angestrebten Lösung des eingangs beschriebenen technischen Problems ist, daß der Folienschlauch über einen Übergangsbereich in Haltelappen übergeht, so daß sowohl zur Faltenbildung neigende Bereiche der Haut des Patienten von dem Übergangsbereich dichtend abgedeckt werden können, als auch kein unmittelbarer Anschluss an die Haut des Patienten erforderlich ist.

Bei allen Ausführungsformen der Erfindung ist auch daran gedacht, den Folienschlauch an seiner dem Übergangsbereich abgewandten axialen Ende dicht zu verschließen, so daß der Fuß bzw. die Hand des Patienten vollständig in dem Schlauch aufgenommen werden können und kein weitere dichtender Anschlussbereich im Bereich des Knöchels und/oder Handgelenks erforderlich ist.

## Patentansprüche

1. Wundversorgungsanordnung mit einer an der eine Wunde umgebenden Haut festlegbaren und zum Herstellen eines die Wunde enthaltenden Wundraums dienenden, zumindest abschnittweise wasserdampfdurchlässigen Folienschlauch aufweisenden Abdeckeinrichtung und einem Sauganschluss, über den ein Unterdruck im Wundraum erzeugt werden kann, **dadurch gekennzeichnet, dass** der Folienschlauch über einen eine quer, insbesondere etwa senkrecht zu einer durch die Schlauchachse und eine die Schlauchachse durchsetzende und senkrecht dazu verlaufende Radialachse aufgespannten Frontalebene verlaufenden Sagittalachse zumindest teilweise umlaufenden Übergangsbereich in einen Haltelappen übergeht, wobei der Haltelappen einen sich ausgehend von dem Übergangsbereich zumindest abschnittsweise in Richtung auf das dem Übergangsbereich abgewandten Ende des Folienschlauchs erstreckenden Halteabschnitt aufweist, der eine in der Frontalebene verlaufende Longitudinalachse teilweise, insbesondere über einen Umfangswinkel von 180° oder weniger und 45° oder mehr umläuft, wobei die Longitudinalachse in einer die Schlauchachse enthaltenden und senkrecht zur Sagittalachse verlaufenden Ebene angeordnet ist und der dem Folienschlauch zugewandte Rand des Übergangsbereichs die Schlauchachse teilweise umläuft.

2. Wundversorgungsanordnung nach Anspruch 1, **dadurch gekennzeichnet, daß** die Schlauchachse und die Longitudinalachse einen spitzen Winkel von vorzugsweise etwa 5° bis 45° miteinander einschließen.

3. Wundversorgungsanordnung nach einem der vorhergehenden Ansprüche, **dadurch gekennzeichnet, daß** der dem Folienschlauch zugewandte Rand des Übergangsbereichs die Schlauchachse über einen Winkelbereich von 180° oder weniger und mehr als 20°, insbesondere mehr als 45°, besonders bevorzugt mehr als 60°, ganz besonders bevorzugt mehr als 90° umläuft.

4. Wundversorgungsanordnung nach einem der vorhergehenden Ansprüche, **dadurch gekennzeichnet, daß** der Übergangsbereich die Sagittalachse über einen Winkelbereich von 180° oder weniger und mehr als 45° umläuft.

5. Wundversorgungsanordnung nach einem der vorhergehenden Ansprüche, **gekennzeichnet durch** zwei von dem Übergangsbereich ausgehende und in sagittaler Richtung voneinander beabstandete Haltelappen.

6. Wundversorgungsanordnung nach einem der vorhergehenden Ansprüche, **dadurch gekennzeichnet, daß** mindestens ein Haltelappen an seinem dem Übergangsbereich abgewandten Ende in einem frei auslaufenden Rand endet.

7. Wundversorgungsanordnung nach einem der vorhergehenden Ansprüche, **dadurch gekennzeichnet, daß** der Folienschlauch auf seiner in Umfangsrichtung dem Übergangsbereich abgewandten Seite in einen die Schlauchachse in Umfangsrichtung über eine Umfangswinkel von 60° oder mehr, vorzugsweise 90° oder mehr, insbesondere etwa 180° umlaufenden Bundbereich übergeht.

8. Wundversorgungsanordnung nach Anspruch 7, **dadurch gekennzeichnet, daß** der Bundbereich in Umfangsrichtung in mindestens einen Haltelappen übergeht, wobei der dem Folienschlauch abgewandte Rand des Bundbereichs vorzugsweise mit einer die Longitudinalachse teilweise umlaufenden Rand des Haltelappens fluchtet.

9. Wundversorgungsanordnung nach einem der vorhergehenden Ansprüche, **dadurch gekennzeichnet, daß** der Folienschlauch, der Übergangsbereich, der Haltelappen und ggf. der Bundbereich ein zusammenhängendes dreidimensionales Flächengebilde bilden.

10. Wundversorgungsanordnung nach Anspruch 9, **dadurch gekennzeichnet, daß** das Flächengebilde von dem dem Übergangsbereich in Richtung der Schlauchachse abgewandten Rand des Folienschlauchs, dem dem Übergangsbereich in Richtung der Schlauchachse abgewandten Rand des Bundbereichs und Rändern des/der Haltelappen vollständig berandet wird.

11. Wundversorgungsanordnung nach Anspruch 9, **dadurch gekennzeichnet, daß** der Folienschlauch an seinem dem Übergangsbereich abgewandten axialen Ende geschlossen ausgeführt ist und das dreidimensionale Flächengebilde von dem dem Übergangsbereich in Richtung der Schlauchachse abgewandten Rand des Bundbereichs und Rändern des bzw. der Haltelappen vollständig berandet wird.

## Claims

1. A wound care arrangement comprising:
a means of covering which includes a film tube, parts of which at least are permeable to water vapour, and which is used to create a wound space enclosing the wound by being fixable to the skin surrounding said wound; and
a suction connection by means of which a vacuum can be created in the wound space,
**characterised in that** the film tube transitions to a retainer flap via a transitional area running at least partly around a sagittal axis extending transversely and in particular approximately perpendicular to a frontal plane that is defined by the tube axis and by a radial axis that intersects the tube axis and extends perpendicularly thereto,
wherein the retainer flap has a retainer portion starting from the transitional area and extending at least in sections towards the end of the film tube facing away from the transitional area, with said retainer portion running, in part at least, around a longitudinal axis extending along the frontal plane, in particular over a circumferential angle of at least 45° and at most 180°, wherein the longitudinal axis is located in a plane containing the tube axis and running perpendicular to the sagittal axis, and the edge of the transitional area facing towards the film tube runs partially around the tube axis.

2. The wound care arrangement according to claim 1, **characterised in that** the tube axis and the longitudinal axis together form an acute angle of preferably approximately 5° to 45°.

3. The wound care arrangement according to one of the preceding claims, **characterised in that** the edge of the transitional area facing towards the film tube runs around the tube axis over an angle range of at least 20° and at most 180°, preferably more than 45°, more preferably more than 60°, and most preferably more than 90°.

4. The wound care arrangement according to one of the preceding claims, **characterised in that** the transitional area runs around the sagittal axis over an angle range of at least 45° and at most 180°.

5. The wound care arrangement according to one of the preceding claims, **characterised by** two retainer flaps which start from the transitional area and are set at a distance from each other in the sagittal direction.

6. The wound care arrangement according to one of the preceding claims, **characterised in that** at least one retainer flap ends in a free edge at its end facing away from the transitional area.

7. The wound care arrangement according to one of the preceding claims, **characterised in that** on the film tube side facing away from the transitional area in the circumferential direction, the film tube transitions to a collar area running circumferentially around the tube axis over a circumferential angle of 60° or more, preferably 90° or more, and in particular approximately 180°.

8. The wound care arrangement according to claim 7, **characterised in that** the collar area transitions to at least one retainer flap in the circumferential direction, wherein the edge of the collar area facing away from the film tube is preferably aligned with an edge of the retainer flap running in part around the longitudinal axis.

9. The wound care arrangement according to one of the preceding claims, **characterised in that** the film tube, the transitional area, the retainer flap and where applicable the collar area form a continuous three-dimensional planar structure.

10. The wound care arrangement according to claim 9, **characterised in that** the planar structure is completely bordered by the edge of the film tube facing away from the transitional area in the direction of the tube axis, the edge of the collar area facing away from the transitional area in the direction of the tube axis and edges of the retainer flap(s).

11. The wound care arrangement according to claim 9, **characterised in that** the film tube is closed at its axial end facing away from the transitional area and the three-dimensional planar structure is completely bordered by the edge of the collar area facing away from the transitional area in the direction of the tube axis and by edges of the retainer flap(s).

## Revendications

1. Dispositif de traitement des plaies comprenant :
un moyen de recouvrement présentant un film tubulaire qui est perméable à la vapeur d'eau, au moins par sections, et permettant de créer un espace englobant la plaie du fait qu'il peut être fixé sur la peau qui entoure ladite plaie, et
un raccord d'aspiration grâce auquel une pression négative peut être produite dans l'espace englobant la plaie ;
**caractérisé en ce que** le film tubulaire se transforme en un pan de retenue par l'intermédiaire d'une zone de transition entourant au moins partiellement un axe sagittal qui est transversal et notamment sensiblement perpendiculaire à un plan frontal défini par l'axe du tube et par un axe radial traversant ledit axe du tube et s'étendant perpendiculairement à ce dernier,
ledit pan de retenue présentant une section de retenue partant de la zone de transition et dirigée, au moins par sections, vers l'extrémité du film tubulaire qui est détournée de ladite zone de transition, laquelle section de retenue entourant partiellement un axe longitudinal situé dans le plan frontal, notamment selon un angle à la circonférence faisant 45° minimum et 180° maximum, ledit axe longitudinal étant disposé dans un plan contenant l'axe du tube et situé perpendiculairement à l'axe sagittal, ledit bord de la zone de transition qui est tourné vers le film tubulaire entourant partiellement ledit axe du tube.

2. Dispositif de traitement des plaies selon la revendication 1, **caractérisé en ce que** l'axe du tube et l'axe longitudinal forment un angle aigu faisant de préférence d'environ 5° à 45°.

3. Dispositif de traitement des plaies selon l'une des revendications précédentes, **caractérisé en ce que** le bord de la zone de transition qui est tourné vers le film tubulaire entoure l'axe du tube sur une plage angulaire de 20° minimum, de préférence de 45° minimum, plus préférablement de 60° minimum, et encore plus préférablement de 90° minimum et 180° maximum.

4. Dispositif de traitement des plaies selon l'une des revendications précédentes, **caractérisé en ce que** la zone de transition entoure l'axe sagittal sur une plage angulaire de 45° minimum et 180° maximum.

5. Dispositif de traitement des plaies selon l'une des revendications précédentes, **caractérisé par** deux pans de retenue partant de la zone de transition et séparés l'un de l'autre dans le sens sagittal.

6. Dispositif de traitement des plaies selon l'une des revendications précédentes, **caractérisé en ce qu'**au moins un pan de retenue se termine par un bord libre à son extrémité détournée de la zone de transition.

7. Dispositif de traitement des plaies selon l'une des revendications précédentes, **caractérisé en ce que** le film tubulaire, sur son côté détourné de la zone de transition dans le sens circonférentiel, se transforme en une zone d'épaulement entourant l'axe du tube dans le sens circonférentiel sur un angle à la circonférence de 60° minimum, de préférence de 90° minimum, et tout préférablement de sensiblement 180°.

8. Dispositif de traitement des plaies selon la revendication 7, **caractérisé en ce que** la zone d'épaulement se transforme, dans le sens circonférentiel, en au moins un pan de retenue, ledit bord de la zone d'épaulement qui est détourné du film tubulaire étant de préférence alignée avec un bord du pan de retenue qui entoure partiellement l'axe longitudinal.

9. Dispositif de traitement des plaies selon l'une des revendications précédentes, **caractérisé en ce que** le film tubulaire, la zone de transition, le pan de retenue et, le cas échéant, la zone d'épaulement forment une structure plane tridimensionnelle cohérente.

10. Dispositif de traitement des plaies selon la revendication 9, **caractérisé en ce que** la structure plane est totalement bordée par le bord du film tubulaire qui est détourné de la zone de transition dans la direction de l'axe du tube, par le bord de la zone d'épaulement qui est détourné de la zone de transition dans la direction de l'axe du tube et par les bords du ou des pans de retenue.

11. Dispositif de traitement des plaies selon la revendication 9, **caractérisé en ce que** le film tubulaire est conçu fermé au niveau de son extrémité axiale détournée de la zone de transition et la structure plane tridimensionnelle est totalement bordée par le bord de la zone d'épaulement qui est détourné de la zone de transition dans la direction de l'axe du tube et par les bords du ou des pans de retenue.
